# EUROPEAN PATENT APPLICATION

(11) **EP 4 092 127 A1**
(43) Date of publication of application: **23.11.2022**
(21) Application number: 22168274.3
(22) Date of filing: 13.04.2022
(51) Int. Cl.: C12P 7/44, C12P 17/02, C12P 41/00, C12N 9/20

(54) **ENZYME-CATALYTIC METHOD FOR THE DIRECT STEREOSELECTIVE SYNTHESIS OF GAMMA-(ACYLOXY) CARBOXYLIC ACIDS**

(30) Priority: 18.05.2021 EP 21174550
(71) Applicant: Tallinn University of Technology, 19086 Tallinn (EE)
(72) Inventor: PARVE, Jaan, 19086 Tallinn (EE); KUDRJASHOVA, Marina, 19086 Tallinn (EE); GATHERGOOD, Nicholas, 19086 Tallinn (EE); PARVE, Omar, 19086 Tallinn (EE)
(74) Representative: Sarap, Margus

(57) **Abstract**

The present invention is a method for the stereoselective straightforward synthesis of gamma-acyloxy carboxylic acids of novel structure starting from racemic gamma-hydroxy carboxylic acid salts that are obtained by alkaline hydrolysis of the racemic gamma-lactones. The salt is acylated by enzyme catalysis in an organic solvent containing an acyl donor and an immobilised lipase, on stirring at room temperature (ca 20 °C) until reaching the conversion rate required. After that, the reaction mixture is acidified and extracted and the product treated with catalytic amount of para-toluenesulfonic acid in toluene during the time required for the relactonisation of the unreacted salt enantiomer. Thereafter, the target gamma-acyloxy carboxylic acid formed from one enantiomer of the starting compound and relactonised gamma-lactone formed from the other, unreacted enantiomer of the starting compound are isolated using a routine separation method, such as distillation, extraction with NaHCO3 water solution followed by acidification or column chromatography.

## Description

### Technical field

The invention belongs - by the method proposed and by the goal of embodiment - to the field of biotechnological organic synthesis. The method invented opens an opportunity to synthesise directly enantiomerically enriched gamma-(acyloxy)carboxylic acids of novel structure. The synthesis starts from racemic gamma-hydroxycarboxylic acid salts which are obtained either by an alkaline hydrolysis of racemic gamma-lactones or starting from any alternative feedstock and using any alternative method. One of the embodiments of the invention is separation of the enantiomers of gamma-lactones. The products synthesised by using the invention are used as feedstocks in polymer, perfumery and pharmaceutical industry.

According to the object of the main embodiment of the invention, which is gamma-valerolactone GVL, the present invention belongs also to the field of refining cellulosic wastes and lignocellulosic biomass, which are the feedstock for the production of gamma-valerolactone GVL. Thus, the invention belongs to the field of wood chemistry as well. [1,2]

### Background Art

In order to sustain the environment, the chemical industry is undergoing reorientation from fossil to renewable feedstocks. One of the suitable strategies is levulinic acid (LA; 4-ketopentanoic acid, 4-oxopentanoic acid) production starting from cellulosic biological feedstocks, which are wood biomass, paper and agricultural wastes not suitable for use in food production [3].

LA is hydrogenised catalytically to racemic 4-hydroxypentanoic acid, which either spontaneously or by acid catalysis lactonises to racemic gamma-lactone.[4,5,6]

Asymmetric hydrogenation of LA in the form of unprotected carboxylic acid by using Ruthenium containing catalysts with chiral ligands has afforded 4-hydroxypentanoic acid and corresponding gamma-valerolactone with only modest enantiomeric excess. The (*S*)-GVL has thus been obtained in 62.4% yield and with enantiomeric excess *e.e.* = 82% [7], but also with *e*.*e*.=78-93% [8].

The 4-keto group of LA esters has been reduced biocatalytically [9-12] as well as chemically with Ruthenium-based asymmetric catalysts [13,14] with high stereoselectivity, affording enantiomerically enriched esters of 4-hydroxypentanoic acid. However, the scalability of the described synthetic methods [9-14] seems to be somewhat problematic.

The enantiomers of different esters and amides of gamma-hydroxycarboxylic acids have been separated by lipase-catalysed acetylation.[15] This method has also been used in combination with simultaneous permanent catalytic racemisation of the unreacted starting compound in order to maximise the yield - the so-called dynamic kinetic resolution. [16] Enantiomerically enriched gamma-lactones have been synthesised by alkaline hydrolysis of enantiomerically enriched esters of gamma-hydroxypentanoic acids as well as of amides and esters of the gamma-(acetyloxy)pentanoic acids, followed in both cases by acidic lactonisation.

Very few works have been dedicated to the synthesis of gamma-(acyloxy)carboxylic acids. [17,18] The syntheses described therein differ substantially from the method that is the present invention. An indirect synthetic approach involving a different number of steps in its different modifications has been used to synthesise several homologs with aliphatic open carbon-chain gamma-(acyloxy)carboxylic acids. These modifications are related by two common central steps: 1) creation of the stereocentre either by a biocatalytic step or by using the Sharpless asymmetric epoxidation method and 2) the Ru(III)-catalysed oxidation of the phenyl group to carboxyl group using NaIO₄ as a stoichiometric oxidiser.

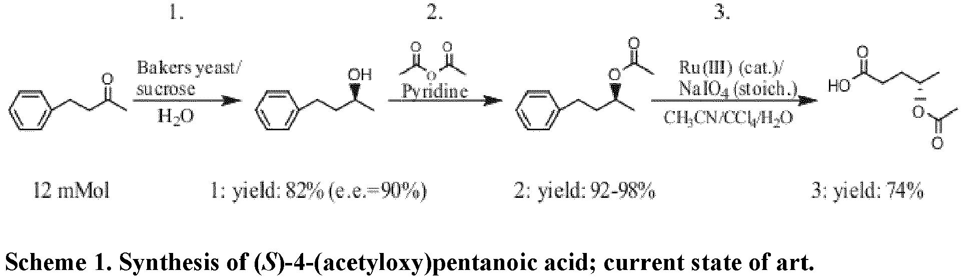

In order to describe the current state of art in comparison with the invention, it is useful to present shortly the most important parameters of the known method [17] in the case of the three-step synthesis of (*S*)-4-(acetyloxy)pentanoic acid (Scheme 1). This target compound can be considered analogous with the target compound of the main embodiument (Example 1) of the invention, (*R*)-4-(propionyloxy)pentanoic acid (*R*)-7.

The current state of art synthesis starts from 2-oxo-4-phenylbutane, which could be currently considered equally accessible to the starting compound of the main embodiment of the present invention (Scheme 2, Example 1), which is racemic gamma-valerolactone (produced from renewable feedstock).

In the first step of the synthesis, the keto group of the starting compound is stereoselectively reduced to a secondary hydroxyl group by fermentation with baker s yeast.

For the fermentation of 12 mmol (1.778 g) of the starting compound, were used, all together, by adding components by portions during five days: sucrose (175 g), baker s yeast (350 g) and water (1.575 litres). The product was extracted from thus formed reaction mixture with 4×0.5 litres of diethyl ether (2.0 litres in total). (S)-2-Hydroxy-4-phenylbutane (*e.e.* 90%) was obtained in 82% yield; 5-10% of 2-phenylethanol was formed as a by-product. The target compound was separated by column chromatography.

The alcohol gained was acetylated using a routine method of chemical synthesis, the acetate was obtained in 92-98% yield and was used in the next step without purification. In the acetylated compound (10 mmol), the phenyl group was oxidised to carboxyl group using a catalytic amount of Ruthenium(III) chloride and a large excess of stoichiometric oxidiser, sodium periodate (0.212 mol; 45.6 g; 21.2-fold excess), the oxidation was performed in a two-phase mixture of tetrachloromethane (40 mL), acetonitrile (40 mL) and water (100 mL).

The product was extracted using 125 mL of dichloromethane; the yield of the target compound was 74%. The description of the isolation of the target compound is insufficient in this publication, the carboxylic acid synthesised was analysed in the form of methyl ester. The esterification was performed using diazomethane, which is a toxic and explosive compound. The overall yield of the target (*S*)-4-(acetyloxy)pentanoic acid was up to 60% (*e.e.* = 90%).

The state of art method described above is clearly not suitable for upscaling, because it necessitates the use of large volumes of solvents and a large excess of reagents, and the separation of an intermediate by column chromatography.

The possibilities of application of racemic and enantiomerically enriched gamma-(acyloxy)carboxylic acids have not been studied extensively because of the exceptionally limited accessibility of the representatives of this class of organic compounds. However, there has been reported their application for the synthesis of enantiomerically enriched gamma-lactones that, in turn, have a very wide field of application. [19,20].

Over the decades, a large number of published works have been devoted to the synthesis of enantiomerically enriched gamma-hydroxycarboxylic acid esters and amides as well as to their acylated derivatives, and also to their transformation to the enantiomerically enriched gamma-lactones. However, despite these efforts, the accessibility of enantiomerically highly enriched (*S*)-GVL and (*R*)-GVL ((*S*)-**1** ja (*R*)-**1**, respectively; Scheme 2) as well as homologous gamma-lactones with longer carbon chain has not been satisfactory.

So far, there has been missing a technically simple, reliable, highly stereoselective and scalable method for the separation of gamma-valerolactone (and of homologous gamma-lactones) enantiomers starting from the racemic mixture. The present invention is intended to fulfill this gap.

### Summary of invention

One of the known ways to synthesise enantiomerically enriched gamma-lactones is the synthesis of corresponding enantiomerically enriched gamma-(acyloxy)carboxylic acids, followed by the hydrolysis and acid catalysed lactonisation of the gamma-hydroxycarboxylic acids gained. [17,18] The present invention is a novel biocatalytic method for the straightforward stereoselective synthesis of gamma-(acyloxy)carboxylic acids starting from racemic salts of gamma-hydroxycarboxylic acids that can be easily synthesised by alkaline hydrolysis of racemic gamma-lactones.

The gamma-hydroxycarboxylic acid salt is acylated stereoselectively by lipase-catalysis in an appropriate organic solvent, the acyl donor used is a vinyl ester or another acyl donor of an alternative structure. Thereafter, the reaction mixture is acidified, which results in relactonisation of the unreacted enantiomer and the formation of the target compound, a stable carboxylic acid that is to be separated. In the main embodiment of the invention (Scheme 2), fractional distillation under reduced pressure (Example 1) is used to separate the target compound; however, other separation methods such as extraction with NaHCO₃ water solution and column chromatography are useable as well.

If the invention is used to acylate homologous gamma-hydroxycarboxylic acid salts of varying carbon chain lengths using Novozym^{®} 435 (an immobilised preparation of *Candida antarctica* lipase B (CALB)) catalysis, it has been found that there takes place a switch of the enantiopreference of CALB from *R* configuration to *S* configuration of the secondary hydroxyl group of the salt. The switch takes place going from gamma-hydroxyhexanoic acid sodium salt (**4**) to gamma-hydroxyheptanoic acid sodium salt (**12**) (Schemes 2 and 3). Accordingly, enantiomerically enriched target compounds of different configuration are obtained.

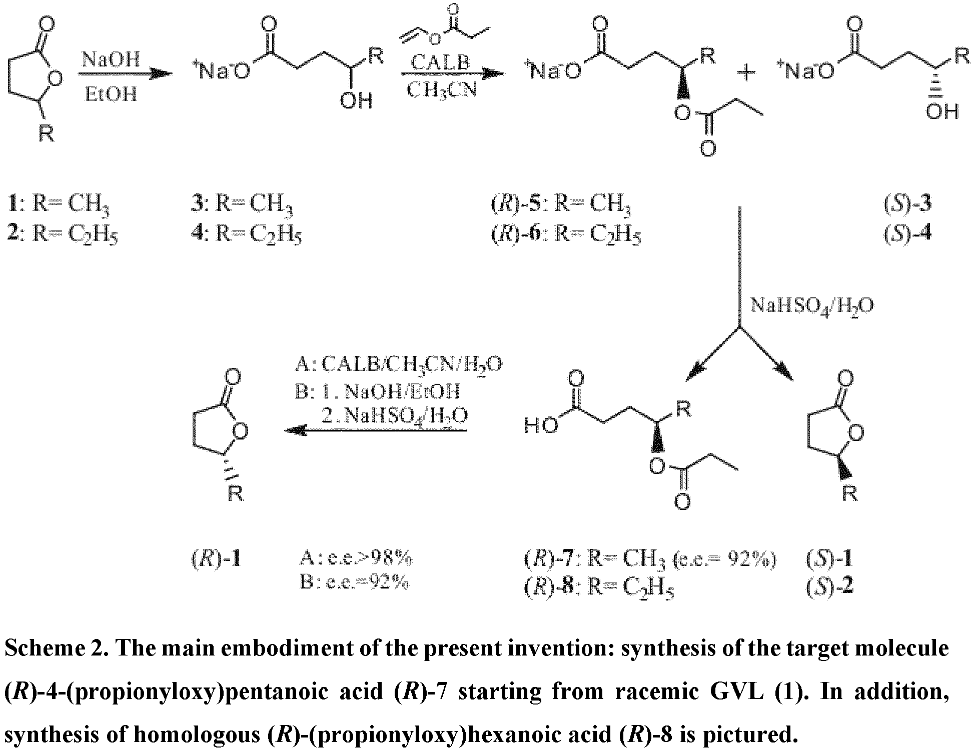

The highest stereoselectivity of the invention (enantiomeric excess *e.e.* of the product was >99.8%) was achieved on acetylation of the -[(1*S*,5*R*)-5-hydroxycyclopent-2-ene-1-yl]ethanoic acid sodium salt (**22**) with CALB as the biocatalyst, followed by, using the same lipase, 4-hydroxyoctanoic acid sodium salt (**13**) and 4-hydroxynonanoic acid sodium salt (**14**) (in both cases *e.e.* >97% was determined for the product). The enantiomeric purity of the product achieved varies depending on the structure of the salt, from moderate to very high. If an implementer of the invention needs to further improve the stereoselectivity, then one option along with further optimisation of the kinetic resolution process is the use of a lower conversion rate. Such a measure may, however, decrease the yield too much. Another dependable way is a second lipase-catalysed treatment of the enantiomerically enriched product, which, as a rule, allows to obtain the product with sufficient enantiopurity (*e*.*e*. >97-99%) in satisfactory yield.

For instance, stereoselective lipase-catalysed hydrolysis of (*R*)-4-(propionyloxy)pentanoic acid with (*e.e.* =92%; Example 1, Scheme 1) obtained from the preparative synthesis, afforded directly highly enantiopure (R)-GVL ((R)-1), *e.e.* >98%), that can be separated by extraction.

An alkaline hydrolysis of the same product (*R*)-**7** (*e.e.* =92%) and following acidic lactonisation affords (*R*)-GVL ((*R*)-**1**) with unchanged enantiomeric purity *(e.e.* =92%). Repeating the synthetic method, that is the invention, starting from the (*R*)-GVL gained affords the target (*R*)-4-(propionyloxy)pentanoic acid ((*R*)-**7**) in satisfactory yield along with high enantiomeric enrichment (*e.e.* > 98%), that is also separable by extraction up to the chemical purity of >98%.

The enantiomeric purity of target gamma-(acyloxy)carboxylic acids was measured by determination of the diastereomeric ratios *d.r.* of ester conjugates prepared by O-alkylation of the carboxylic acids with a chiral derivatising agent (1*R*)-menthyl bromoacetate (Schemes 5 and 6); *d.r.* was determined by ¹H NMR spectroscopy. The enantiomeric purity of the product (*R*)-4-(propionyloxy)pentanoic acid ((*R*)-7), gained in the main embodiment of the invention (Example 1), was additionally measured by HPLC of the *p*-bromophenacyl esters **31**, that were synthesised by a routine method (Scheme 7; Example 8; HPLC data, conditions and retention times are provided); enantiomeric ratio e.r. of target compounds was determined also by chiral gas liquid chromatography of gamma-lactones (a method well known in the art) obtained via hydrolysis and lactonization of target gamma-(acyloxy)carboxylic acids.

Upon applying the invention on the acylation of the sodium salt obtained from the main object of the invention - which is racemic gamma-valerolactone - a significant positive effect was observed, which is, that the acylation of 4-hydroxypentanoic acid sodium salt in an organic solvent involving CALB as the biocatalyst and vinyl propionate as the acyl donor was found to be an exceptionally intense process compared to homologous compounds. The reaction rate is at least 10-100 (or even more) times higher depending on the structure of the homolog. In order to convert (up to conversion of 40-50%) the sodium salt obtained from one mol of GVL (100.12 g) in 22 hours, 2.0 grams of Novozym^{®} 435 are needed; the bioacatalyst is repeatedly reusable.

The best stereoselectivity of the lipase-catalysed acylation of the 4-hydroxypentanoic acid sodium salt obtained from racemic GVL (conversion was 40%, *e.r.* of the product was 96.1/3.9 (*R*/*S*)) was achieved for the target compound of novel structure, (*R*)-4-(propionyloxy)pentanoic acid (*R*)-7; the synthesis was performed in acetonitrile and catalysed by Novozym^{®} 435 which is *Candida antarctica* lipase B immobilized via interfacial activation on a resin which is macroporous support formed by poly(methyl methacrylate) crosslinked with divinylbenzene. In other examples provided herein, the target compounds obtained starting from homologous gamma-lactones are compounds of novel structure as well. The term "novel structure" means that, as far as we know, neither a compound of this certain structure nor its synthesis have been described before in literature and that the (novel) structure differs from any of the structures described in the literature by chemical or stereochemical structure or by both of them.

The compounds of novel structure synthesised by using the present invention are the following compounds (their syntheses are described in the examples provided in the current text):
1) (*R*)-4-(propionyloxy)pentanoic acid (*R*)-**7;** a synthesis of an analogous compound (*S*)-4-(acetyloxy)pentanoic acid has been reported [17] and is analysed herein above as a state of art method (Scheme 1);
2) (*R*)-4-(propionyloxy)hexanoic acid (*R*)-**8**; there has been described an 8 step synthesis of an analogous compound (*R*)-4-(acetyloxy)hexanoic acid which involves among others Wittig condensation and Sharpless asymmetric epoxidation steps; steps requiring chromatographic separation of the products have been used; the method is clearly more complicated and resource consuming compared to the invention;
3) (*S*)-4-(acetyloxy)heptanoic acid (*S*)-**18**; any syntheses of neither this compound nor of its stereoisomers or analogs has been described in literature;
4) (*S*)-4-(acetyloxy)octanoic acid (*S*)-**19**; the synthesis of this compound has been described in literature, albeit as a constituent of racemic mixture; the method described involves catalytic oxidation of the phenyl group in the final step [18];
5) (*S*)-4-(acetyloxy)nonanoic acid (*S*)-**20**; any syntheses of this compound nor of its stereoisomers or analogs has been described in literature;
6) 2-[(1*S*,5*R*)-5-acetyloxycyclopent-2-ene-1-yl]ethanoic acid (1*S*,5*R*)-**24**; no synthesis of a compound with exactly identical structure was found in literature.

It should be added, in order to explain the nature of the invention, that aliphatic gamma-hydroxycarboxylic acids are not stable compounds. They lactonise spontaneously, and especially smoothly and quantitatively under acid catalysis, thereby liberating a water molecule. An efficient reverse reaction is alkaline hydrolysis of gamma-lactones, which occurs smoothly in ethanol by means of sodium hydroxide or any other alkali metal hydroxide allowing to decyclise a gamma-lactone quantitatively. A gamma-hydroxycarboxylic acid alkali metal salt obtained in the form of a solid upon evaporation is a stable compound which relactonises promptly upon acidification and acid catalysis. A direct enzymatic stereodiscriminative treatment of gamma-lactones by hydrolysis directed at attacking the carbonyl electrophilic centre of the ester functional group has not been sufficiently stereoselective nor smooth (*e*.*e*. of the product was 70%). [21]

The invention, on the contrary, is based on an alternative way which has still not been used. The hydroxyl group of an open-chain hydroxycarboxylic acid salt obtained by alkaline hydrolysis of a gamma-lactone is acylated stereoselectively by lipase-catalysis in an organic solvent. The following acidification of the crude acylation product affords stabile gamma-(acyloxy)carboxylic acid along with relactonised unreacted enantiomer, which are easily separable by routine separation technologies.

Gamma-valerolactone (GVL) is a sustainable platform chemical with unique properties, that is already the starting point of the syntheses of several valuable compounds. The invention adds to the already existing ones a novel, highly intensive biocatalytic process for the stereodiscriminative refining of racemic GVL. The invention enables to synthesise (*R*)-gamma-(acyloxy)pentanoic acids of novel structure with required enantiomeric purity starting from racemic GVL. In order to demonstrate the scope of the invention there are also included examples of the stereodiscriminative derivatisation of the salts obtained from higher homologs of chiral gamma-lactones and additionally also an example of gamma-lactones with a more complex, bicyclic structure.

### Description of embodiments

To determine the scope and limitations of the applicability of the invention, trials with the first five homologous chiral gamma-lactones and with bicyclic so-called *Grieco lactone*: 2-oxabicyclo[3.3.0]oct-6-en-3-one were performed. In order to reach an acceptable result, the derivatisation of the first member of the row of homologs, GVL, was optimised more extensively compared to those starting from the higher homologs which are presented herein in the examples. For GVL, the influence of the most important factors on the stereoselectivity and velocity of the reaction was tested. These factors are: 1) the choice of lipase, its stereopreference along the row of the homologs and loading vs. certain time of incubation; 2) the influence of the salt counterion; 3) the structure of the acyl moiety to be lipase-catalytically transferred); 4) organic solvent used as reaction medium; 5) temperature.

It was found, that all the factors listed have significant influence either on the reaction velocity or on stereoselecitvity or on both of them. The most preferable reaction conditions in the case of 4-hydroxypentanoic acid salt derived from racemic Gamma-valerolactone (GVL) are: 1) the lipase is *Candida antarctica* lipase B (CALB) immobilised on a polymer carrier and marketed as Novozym^{®} 435; the configuration of 4-hydroxypentanoic acid sodium salt preferred by CALB is R; 2 mg of Novozym^{®} 435 per 1 mmol of the sodium salt per 22 hours of incubation in acetonitrile affording 40% conversion; 2) sodium is the preferred salt counterion compared to lithium and potassium; 3) propionyl is preferred compared to acetyl and butyryl; 4) acetonitrile is the preferred reaction medium; 5) optimal temperature is +20°C.

In general, the same reaction conditions are also preferred in the case of salt derived from hexano-1,4-lactone, whereas the reaction velocity was approximately 20-fold slower.

For all other four lactones tested: heptano-1,4-lactone, octano-1,4-lactone, nonano-1,4-lactone and 2-oxabicyclo[3.3.0]oct-6-en-3-one (*Grieco lactone*) the highest stereoselectivity was achieved in chloroform (the reaction velocity was approximately 100-200-fold slower compared to acylation of 4-hydroxypentanoic acid sodium salt); in some examples alternative solvents are used.

The on-line monitoring of the reaction did not succeed because of the multiphase nature of the system which contains several different solid phases which are changing during the reaction. Instead, the end product was analysed: both, the conversion rate and enantiomeric purity of the product were determined by means of ¹H NMR spectroscopy. The conversion rate was determined by the molar ratio of the product vs. gamma-lactone recovered; while the *e.r.* of the 4-(acyloxy)carboxylic acid gained was determined using NMR - by determining diastereomeric ratio of the ester conjugates synthesised by O-alkylating the carboxyl group of the product with a chiral derivatising agent, (1*R*)-menthyl bromoacetate; or by chiral HPLC of the corresponding *para*-bromophenacyl esters or by chiral gas liquid chromatography of gamma-lactones obtained by alkaline hydrolysis of the product followed by acidic relactonisation. The yields and sterochemical purity of the products are given in the examples of the synthesis.

The present invention is a method for the straightforward stereoselective synthesis of gamma-(acyloxy)carboxylic acids from the salts of racemic gamma-hydroxycarboxylic acids, which are gained by alkaline hydrolysis of gamma-lactones or by any other method. The salt is acylated by enzyme-catalysis in an organic solvent containing also an acyl donor (the lipase substrate) and an immobilised lipase and by stirring the reaction mixture at room temperature approximately +20 °C until the proper conversion rate is reached. Thereafter the enzyme is filtered off and all volatile material is evaporated on a rotary evaporator, the reaction mixture is acidified with NaHSO4 water solution, extracted and relactonised additionally in the case of homologs with longer than C5 carbon chain by stirring with catalytic amount of *para-*toluenesulfonic acid in organic solvent containing anhydrous sodium sulphate. The target compound formed from one enantiomer and the gamma-lactone relactonised from the other unreacted enantiomer are separated by using a routine separation method, which is either distillation, extraction with NaHCO₃ water solution, or column chromatography.

The application of the invention in the case of the first five homologous gamma-lactones, the target compounds of the most proper structure, in the terms of stereoselectivity, were novel structures. Moving from hexa-1,4-lactone to hepta-1,4-lactone the stereopreference of CALB swithced from *R*-configuration to *S*. Accordingly, the absolute configuration of the corresponding gamma-(acyloxy)carboxylic acids gained by the invention is different.

The steroselectivity of the acylation of the sodium salt corresponding to GVL was highest for the synthesis of (*R*)-4-(propionyloxy)pentanoic acid (*e.e.* of the product was 92%). The lipase-catalysed stereoselective hydrolysis of this product (using Novozym^{®} 435 in acetonitrile/water) afforded (*R*)-GVL in one step with high enantiomeric purity (e.e. >98%). Starting from racemic GVL and applying the necessary conditions allowed the synthesis of racemic gamma-acyloxypentanoic acids as well.

**The protocols of the stereoselective biocatalytic syntheses of enantiomerically enriched gamma-(acyloxy)carboxylic acids.**

### Example 1. The synthesis of (R)-4-(propionyloxy)pentanoic acid (R)-7.

γ-Valerolactone (GVL) **1** (30.04 g; 28.61 mL; 300 mmol) was added to NaOH solution in ethanol (1M; 300 mL) and the solution was stirred at 20 °C for one hour. The solution was evaporated and further evacuated until constant weight of the solid salt (41.98 g) was reached. Acetonitrile (300 mL) was added to the sodium salt of 4-hydroxy pentanoic acid gained, followed by the substrate, vinyl propionate (90.12 g; 98.4 mL; 0.9 mol; 3 equiv.), and the biocatalyst Novozym^{®} 435 (600 mg). The mixture was stirred for 22 hours at 20 °C, after which it was acidified by adding NaHSO₄ water solution (2M; 165 mL; 1.1 equiv.). The mixture was stirred for 2 hours, then filtered through a glass filter equipped with a layer of filter aid Hyflo^{®} Super Cel^{®}. The organic volatile material was evaporated from the filtrate and the residue was extracted with EtOAc (Ethyl Acetate, 1×200 mL; 2x 100 mL); the extract was washed with brine (2x20 mL), dried over anhydrous sodium sulphate, filtered and evaporated. In this case - an especial relactonisation step catalysed by *para*-toluenesulfonic acid in toluene containing a drying agent was not necessary because formation of gamma-valerolactone is energetically favoured and occurrs promptly under the conditions of this synthesis.

The components of the crude product (63.2 g) were separated by distillation under reduced pressure. The following four fractions were taken:
1) 10.22 g (42-68°C / 9 mm Hg) of material consisting mainly of propionic acid.
2) 14.56 g (68-70°C / 9 mm Hg) of (*S*)-GVL (*S*)-1 (chemical purity >90%, propionic acid impurity).
3) 2.81 g (62-128°C / 4 mm Hg) of a mixture of (*S*)-GVL and (*R*)-4-(propionyloxy)pentanoic acid (*R*)-7.
4) 16.31 g (128-129°C / 4 mm Hg) of (*R*)-7 (yield: 31.2%; chemical purity: >98%; *e*.*r*. = 96.1/3.9).

Characterization of (*R*)-7: ¹H NMR (800 MHz, CDCl₃) δ 10.8 (bs, 1H, COOH), 4.95 (dqd, *J* = 7.4, 3x6.3, 5.3 Hz, 1H, H-4), 2.40 and 2.39 (m, 2H, H-2), 2.31 (m, *J* = 3x 7.6 Hz, 2H, Pr H-2), 1.89 (m, 2H, H-3), 1.23 (d, *J =* 6.3 Hz, 3H, H-5), 1.12 (t, *J* = 7.6 Hz, 3H, Pr H-3). ¹³C NMR (201 MHz, CDCl₃) δ 179.19 (C-1), 174.13 (Pr C-1), 69.67 (C-4), 30.60 (C-3), 30.07 (C-2), 27.76 (Pr C-2), 19.81 (C-5), 9.05 (Pr C-3). [α]_{D}²⁰ -14.8 (c 7.0; EtOAc; e.r. = 96.1/3.9); IR (neat, cm-1): 457, 649, 808, 869, 905, 931, 1003, 1083, 1134, 1192, 1281, 1338, 1380, 1421, 1463, 1713, 1734, 2983, 3174.

### Example 2. The synthesis of (R)-4-(propionyloxy)hexanoic acid (R)-8.

Racemic hexano-1,4-lactone 2 (1.142 g; 1.109 mL; 10 mmol) was added on stirring to NaOH solution in EtOH (1M; 10 mL). The solution was stirred for one hour at 20 °C. The mixture was evaporated on a rotary evaporator and the flask was evacuated up to constant weight.

Acetonitrile (20 mL) was added to the salt, followed by vinyl propionate (4.57 g; 4.97 mL; 40 mmol) and the biocatalyst Novozym^{®} 435 (0.20 g). The mixture was stirred at 20 °C for 44 hours. Thereafter, the mixture was filtered, the flask and the filter cake were washed thoroughly (aided with mechanical treatment, while the flow is temporarily minimized) with EtOH (Ethanol, 20 mL + 15 mL) in order to dissolve the sodium carboxylates on the filter. The filtrate was evaporated on a rotary evaporator and the flask was further evacuated until constant weight of the residue was reached. Acetonitrile (30 mL) was added to the residue followed by NaHSO₄ water solution (1M; 15 mL) and the mixture was shaken until all solid material in the flask was dissolved; the solution was stirred at 20 °C for two hours (under acidic conditions, pH < 3). Acetonitrile was then evaporated on a rotary evaporator and EtOAc (70 mL) was added to the residue to extract the organic material, the mixture was shaken thoroughly and the water layer separated. The extract was washed with brine (2 × 10 mL) and dried over anhydrous sodium sulphate, filtered and evaporated on a rotary evaporator. The crude mixture was dissolved in toluene (40 mL), a catalytic amount (5 mg) of *para*-toluenesulfonic acid was added and the mixture was stirred for 12 hours. Then the mixture was evaporated and the target compound was separated by column chromatography (eluent: CHCl₃/EtOH 100/3) to afford 207 mg (yield: 11.0%) of (*R*)-4-(propionyloxy)hexanoic acid (*R*)-**8** with conversion of 16.7%; (*e.r.* 88.8/11.2) was measured by ¹H NMR of diastereomeric (1*R*)-menthyl ester conjugates (*R*)-**26**/(*S*)-**26** obtained via O-alkylation of the carboxyl group of the target compound with (*1*R)-menthyl bromoacetate.

Characterization of (*R*)-4-(propionyloxy)hexanoic acid (*R*)-**8**: ¹H NMR (800 MHz, CDCl₃) δ 10.4 (bs, 1H, COOH), 4.85 (dddd, *J* = 8.5. 6.7, 5.8, 4.0 Hz, 1H, H-4), 2.37and 2.36 (m, 2H, H-2), 2.31 (q, *J* = 3x 7.6 Hz, 2H, Pr H-2), 1.93 (dddd, *J* = 14.3, 8.7, 7.1, 4.0 Hz, 1H, H-3), 1.85 (dtd, *J* = 14.3, 2x8.4, 6.6 Hz, 1H, H-3), 1.58 (m, 2H, H-5), 1.13 (t, *J* = 7.6 Hz, 3H, Pr H-3), 0.884 (t, *J* = 7.5 Hz, 3H, H-6). ¹³C NMR (201 MHz, CDCl₃) δ 179.1 (C-1), 174.3 (Pr C-1), 74.1 (C-4), 30.0 (C-2), 28.4 (C-3), 27.7 (Pr C-2), 26.9 (C-5), 9.4 (C-6), 9.2 (Pr C-3).

### Example 3. The synthesis of (S)-4-(acetyloxy)heptanoic acid (S)-18.

Racemic heptano-1,4-lactone **9** (385 mg; 0.387 mL; 3 mmol) was added on magnetic stirring to the solution of NaOH in EtOH (1M; 3 mL). The solution was stirred at 20 °C for one hour. The mixture was evaporated on a rotary evaporator; the flask containing the residual 4-(hydroxy)heptanoic acid sodium salt **12** was further evacuated until a constant weight was reached. EtOAc (20 mL) was added to the sodium salt followed by vinyl acetate (1.290 g; 1.386 mL; 15 mmol) and the biocatalyst Novozym^{®} 435 (0.40 g). The mixture was stirred at 20 °C for 72 hours. Thereafter the mixture was filtered, the flask washed with ethanol (15 mL) that was further used for washing the filter cake, the washing was repeated with an additional amount of EtOH (15 mL) in order to dissolve all carboxylates on the filter.

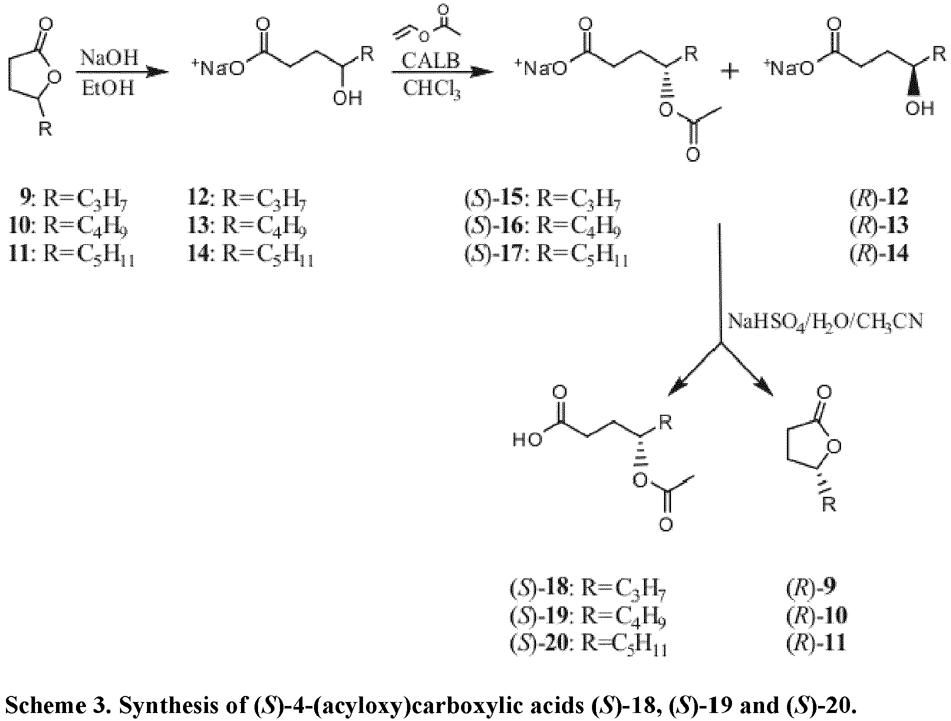

The filtrate was evaporated and the flask was evacuated to remove EtOH, until constant weight of the salt was reached. Acetonitrile (20 mL) was added to the crude product followed by NaHSO₄ water solution (1M; 6 mL); the mixture was shaken until total dissolution of the solid product. The solution was stirred for two hours at 20 °C. Acetonitrile was evaporated from the water solution, EtOAc (60 mL) was added, the mixture was shaken thoroughly and the water layer was separated. The extract was washed with brine (2 × 10 mL) and dried over anhydrous sodium sulphate, filtered and evaporated. The crude mixture was dissolved in toluene (40 mL), a catalytic amount (5 mg) of *para*-toluenesulfonic acid was added and the mixture was stirred for 12 hours. Then the mixture was evaporated and the molar ratio of (*S*)-4-(acetyloxy)heptanoic acid (*S*)-**18** vs. recovered (*R*)-heptano-1,4-lactone (*R*)-**9** in the crude product was determined by ¹H NMR: the result (45.6%) was defined as the conversion rate of the enzymatic acetylation. The compounds were separated by column chromatography (eluent: CHCl₃/EtOH 100/2); (*R*)-**9** (184 mg) and the target compound (*S*)-**18** (191 mg; yield: 36.6%) were gained. (*S*)-**18** was esterfied with (1*R*)-menthyl bromoacetate and the ratio of the diastereomers **27** (96.2/3.8, *S*/*R*) was measured by ¹H NMR.

Characterization of (*S*)-4-(acetyloxy)heptanoic acid (*S*)-**18**: TLC: R_{f} = 0.24 (eluent: PE/EtOAc/EtOH 4/2/0.1); ¹H NMR (800 MHz, CDCl₃) δ 11.0 (bs, 1H, COOH), 4.93 (dddd, *J* = 8.5, 7.5, 5.3, 3.9 Hz, 1H, H-4), 2.40 and 2.39 (m, 2H, H-2), 2.05 (s, 3H, Ac), 1.94 (m, 1H, H-3), 1.84 (m, 1H, H-3), 1.58 and 1.49 (m, 2H, H-5), 1.33 (m, 2H, H-6), 0.91 (t, *J* = 7.4 Hz, 3H, H-7). ¹³C NMR (201 MHz, CDCl₃) δ 179.04 (C-1), 170.96 (Ac), 72.95 (C-4), 36.11 (C-5), 29.98 (C-2), 28.80 (C-3), 21.11 (Ac), 18.47 (C-6), 13.88 (C-7). Structure of this compound was also confirmed by 1JCC values from ¹³C satellites in proton decoupled ¹³C NMR spectrum. 1*JCC* values in Hz from C-1 to C-7: 55.8, 35.5, 39.1, 39.1, 34.5, 34.7. In Ac 1JCC = 59.5 Hz.

### Example 4. The synthesis of (S)-4-(acetyloxy)octanoic acid (S)-19.

Racemic octano-1,4-lactone **10** (711 mg; 0.725 mL; 5 mmol) was added on stirring to NaOH solution in EtOH (1M; 5 mL). The solution was stirred for one hour at 20 °C. The mixture was evaporated and the flask further evacuated until a constant weight was reached. CHCl₃ (20 mL) was added to the salt, followed by vinyl acetate (2.151 g; 2.31 mL; 25 mmol) and the biocatalyst Novozym^{®} 435 (0.50 g). The mixture was stirred at 20 °C for 96 hours. Thereafter it was filtered, the flask and filter cake were washed with EtOH (20 mL + 15 mL) until only granules of the biocatalyst remained on the filter. The filtrate was evaporated on a rotary evaporator and the flask was further evacuated in order to separate EtOH completely. Acetonitrile (20 mL) was added to the crude product, followed by NaHSO₄ water solution (2M; 4 mL); the mixture was shaken thoroughly until all solids were dissolved; the mixture was then stirred for two hours at 20 °C. Acetonitrile was evaporated from the mixture on a rotary evaporator. EtOAc (60 mL) was added to the residual material, the mixture was shaken and the water layer was separated. The extract was washed with brine (2 × 10 mL), dried over anhydrous sodium sulphate, filtered and evaporated. The crude mixture was dissolved in toluene (40 mL), a catalytic amount (5 mg) of *para*-toluenesulfonic acid was added and the mixture was stirred for 12 hours. Then the mixture was evaporated and the ratio of (*S*)-4-(acetyloxy)octanoic acid (*S*)-**19** vs. recovered (*R*)-(+)-octano-1,4-lactone (*R*)-**10** in the crude product was determined by ¹H NMR, the result (46.1%) was defined as conversion rate of the enzymatic acetylation. The product was separated by column chromatography (eluent: CHCl₃/EtOH 100/2) to afford 361 mg of (*R*)-**10** and 390 mg of (*S*)-4-(acetyloxy)octanoic acid (*S*)-**19** (yield: 38.6%). The target compound gained was esterfied with the chiral derivatizing agent (1*R*)-menthyl bromoacetate and diastereomeric esters **28** were analyzed by ¹H NMR: *d.r.* (*e.r.*) = 99/1 (*S*/*R*).

Characterization of (S)-19: TLC: R_{f}=0.50 (eluent: pethroleum ether/EtOAc/MeOH 8/4/1). ¹H NMR (800 MHz, CDCl₃) δ 11.0 (bs, 1H, COOH), 4.90 (dddd, *J* = 8.4. 7.4, 5.5, 3.9 Hz, 1H, H-4), 2.39 and 2.38 (m, 2H, H-2), 2.04 (s, 3H, Ac), 1.94 (m, 1H, H-3), 1.83 (m, 1H, H-3), 1.57 and 1.50 (m, 2H, H-5), 1.27 (m, 2H, H-6), 1.29 (m, 2H, H-7), 0.88 (t, *J* = 7.2 Hz, 3H, H-8). ¹³C NMR (201 MHz, CDCl₃) δ 179.43 (C-1), 170.99 (Ac), 73.20 (C-4), 33.65 (C-5), 30.04 (C-2), 28.74 (C-3), 27.29 (C-6), 22.46 (C-7), 21.08 (Ac), 13.91 (C-8). [α]_{D}²⁰ +5.2 (c = 13; EtOAc).

### Example 5. The synthesis of (S)-4-(acetyloxy)nonanoic acid (S)-20.

Racemic nona-1,4-lactone **11** (781 mg; 0.805 mL; 5 mmol) was added on stirring to NaOH solution in EtOH (1M; 5 mL). The mixture was stirred at 20 °C for one hour. The mixture was evaporated on a rotary evaporator; the flask containing residual sodium salt was further evacuated until a constant weight was achieved. EtOAc (20 mL) was added to the γ-nonanoic acid sodium salt, followed by vinyl acetate (2.151 g; 2.31 mL; 25 mmol) and the biocatalyst Novozym^{®} 435 (0.50 g). The mixture was stirred at 20 °C for 120 hours. The mixture was filtered and the filter cake was washed thoroughly with ethanol (20 mL + 15 mL). The filtrate was evaporated and the flask was further evacuated until a constant weight was reached. Acetonitrile (20 mL) was added to the solid residue, followed by NaHSO₄ water solution (1M; 8 mL). The mixture was shaken until total dissolution of the solid material and stirred 2 hours at 20 °C. Acetonitrile was evaporated on a rotary evaporator, EtOAc (60 mL) was added, the mixture was shaken and the water layer separated. The extract was washed with brine (2 × 10 mL), dried over anhydrous sodium sulphate and evaporated. The crude mixture was dissolved in toluene (40 mL), a catalytic amount (5 mg) of *para*-toluenesulfonic acid was added and the mixture was stirred for 12 hours. Then the conversion rate (44.2%) of the reaction was measured by ¹H NMR as the molar ratio of the target (*S*)-4-(acetyloxy)nonanoic acid (*S*)-**20** and the recovered (*R*)-nona-1,4-lactone (*R*)-**11** in the crude product. Afterwards, 40 mL of EtOAc was added to the solution and the target (*S*)-4-(acetyloxy)nonanoic acid (*S*)-**20** was extracted with 3x25 mL of NaHCO₃ water solution (0.1 M), the water solution of sodium salt (*S*)-**17** was gained. The residual organic solution, after washing with brine (2x15 mL) drying over anhydrous sodium sulphate, filtering and evaporation afforded 394 mg of the homogeneous lactone (*R*)-**11**. The water extract was washed with 2x25 mL of EtOAc, then it was acidified with 6 mL of NaHSO₄ 2M solution and the target pure carboxylic acid was extracted with 3x30 mL of EtOAc, the extract was washed with brine (2x15 mL) and dried over anhydrous sodium sulphate, filtered and evaporated to afford 412 mg of the target (*S*)-**20** (yield: 38.0%). Enantiomeric ratio e.r. of (*S*)-**20** was measured by ¹H NMR of the diastereomeric ratio of ester conjugates **29** obtained by O-alkylation of the carboxylic acid with (1*R*)-menthyl bromoacetate: 98.5/1.5 (*S*/*R*).

Characterization of (*S*)-**20**: TLC: Rf=0.54 (eluent: pethroleum ether/EtOAc/MeOH 8/4/1); ¹H NMR (800 MHz, CDCl₃) δ 11.1 (bs, 1H, COOH), 4.90 (dddd, *J* = 8.4. 7.4, 5.5, 3.9 Hz, 1H, H-4), 2.39 and 2.38 (m, 2H, H-2), 2.05 (s, 3H, Ac), 1.95 (m, 1H, H-3), 1.84 (m, 1H, H-3), 1.57 and 1.51 (m, 2H, H-5), 1.27 (m, 2H, H-7), 1.29 (m, 4H, H-6,8), 0.88 (t, *J* = 7.1 Hz, 3H, H-9). ¹³C NMR (201 MHz, CDCl₃) δ 178.87 (C-1), 170.94 (Ac), 73.20 (C-4), 33.96 (C-5), 31.57 (C-7), 29.96 (C-2), 28.78 (C-3), 24.86 (C-6), 22.49 (C-8), 21.13 (Ac), 13.99 (C-9).

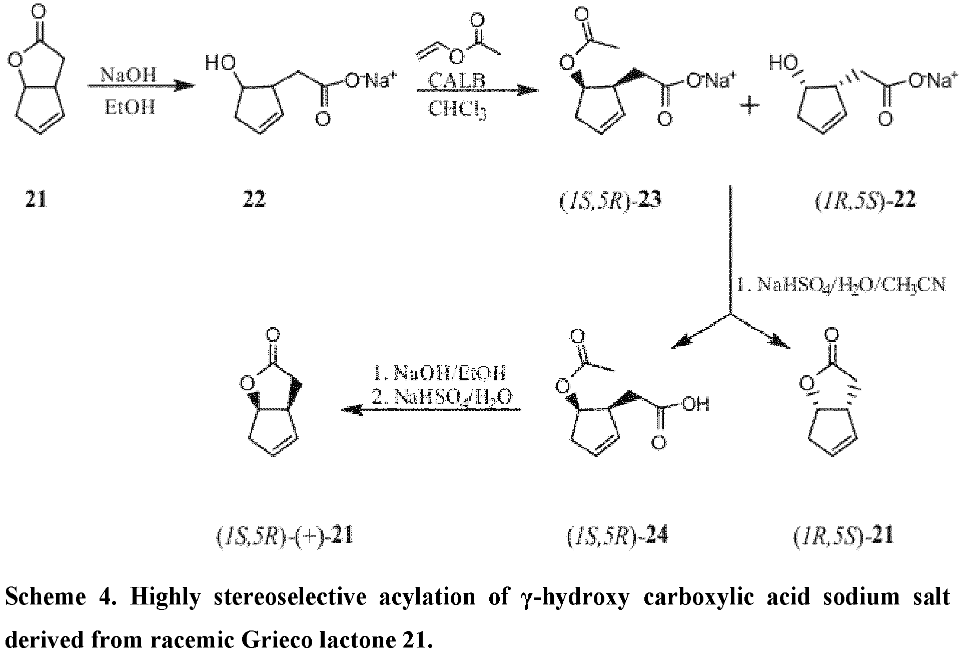

### Example 6. The synthesis of [(1S,5R)-5-(acetyloxy)cyclopent-2-en-1-yl]acetic acid (1S,5R)-24 (a derivative/precursor of (+)-Grieco lactone (1S,5R)-(+)-21).

Racemic 2H,3H,3aH,6H,6aH-cyclopenta[b]furan-2-one (*Grieco lactone*) **21** (621 mg; 5 mmol) was added to NaOH solution in EtOH (1M; 5 mL). The solution was stirred for one hour at 20 °C. The solution was evaporated on a rotary evaporator; the flask was further evacuated until constant weight of the sodium salt was reached. CHCl₃ (15 mL) was added to the salt, followed by vinyl acetate (2.151 g; 2.31 mL; 25 mmol) and the biocatalyst Novozym^{®} 435 (0.50 g). The mixture was stirred at 20 °C for 120 hours. The mixture was filtered through a glass filter covered with filter aid, the flask and the filter cake were washed with EtOH (20 mL + 15 mL). The filtrate was evaporated and the flask was evacuated to constant weight. Acetonitrile (20 mL) and NaHSO₄ water solution (1M; 8 mL) were added to the residual solid material obtained from the filtrate and the mixture was shaken until total dissolution of the solid material. Acetonitrile was evaporated from the solution, and the residual material was extracted with EtOAc (60 mL). The extract was washed with brine (2 × 10 mL), dried over anhydrous sodium sulphate, filtered and evaporated. The crude mixture was dissolved in toluene (40 mL), a catalytic amount (5 mg) of *para*-toluenesulfonic acid was added and the mixture was stirred for 12 hours. The mixture was evaporated and the target compound was separated by column chromatography (eluent: CHCl₃/EtOH 100/2) to afford 320 mg of [(1*S*,5*R*)-5-acetyloxy)cyclopent-2-en-1-yl]acetic acid (1*S*,5*R*)-**24** (yield: 34.7%) along with 392 mg of the recovered (-)-lactone (1*R*,5*S*)-**21**. A sample of (1*S*,5*R*)-**24** was hydrolyzed to the (+)-lactone and analysed using chiral gas liquid chromatography, a method known in the art (minor enatiomer was detected on the level of 0.1% as compared with authentic sample).

Characterization of [(1*S*,5*R*)-5-(acetyloxy)cyclopent-2-en-1-yl]acetic acid (1*S*,5*R*)-**24**: TLC: R_{f}=0.34 (Eluent: Petroleum ether/EtOAc/MeOH 8/4/1); ¹H NMR (800 MHz, CDCl₃) δ 5.77 (dq, *J* = 6.0, 3x2.3 Hz, 1H, H-3), 5.69 (td, *J* = 2x6.7, 3.2 Hz, 1H, H-5), 3.29 (m, 1H, H-1), 2.75 (ddtd, *J* = 17.5, 6.7, 2x2.3, 1.4 Hz, 1H, H-4), 2.57 (dd, *J* = 16.5, 8.2 Hz, 1H, H-6), 2.47 (dd, *J* = 16.5, 7.4 Hz, 1H, H-6), 2.36 (dddt, *J* = 17.5, 3.2, 2.3, 2x1.7 Hz, 1H, H-4), 2.03 (s, 3H, Ac). ¹³C NMR (201 MHz, CDCl₃) δ 178.97 (C-7), 170.77 (Ac C-1), 131.65 (C-2), 128.95 (C-3), 74.27 (C-5), 43.88 (C-1), 39.03 (C-4), 33.25 (C-6), 20.89 (Ac C-2).

### Example 7. The synthesis of 2-1[(1R,2S,5R)-5-methyl-2-(propan-2-yl)cyclohexyl]oxyl-2-oxoethyl (4R)-4-(propionyloxy)pentanoate ((R)-25 with minor diastereomer (S)-25, Scheme 5). This is chiral derivatization of (R)-4-(propionyloxy)pentanoic acid (R)-7.

(1*R*)-Menthyl bromoacetate (83 mg; 0.3 mmol) in EtOAc (5 mL) was added to (*R*)*-4-*(propionyloxy)pentanoic acid (*R*)-7 (35 mg; 0.2 mmol) in acetonitrile (5 mL) followed by DIPEA (65 mg, 87 µL, 0.50 mmol). Reaction mixture was stirred overnight at RT. The mixture was evaporated and the residual material dissolved in EtOAc (50 mL); the solution was washed with sodium bicarbonate solution (0.5 M; 2x5 mL) and brine (2x5 mL), dried over anhydrous Na₂SO₄, filtered and evaporated. The product was purified by flash chromatography (PE/EtOAc 10/0.5) to afford 69 mg of the target ester conjugates (*R*)-**25**/(*S*)-**25** (y.: 93.4%).

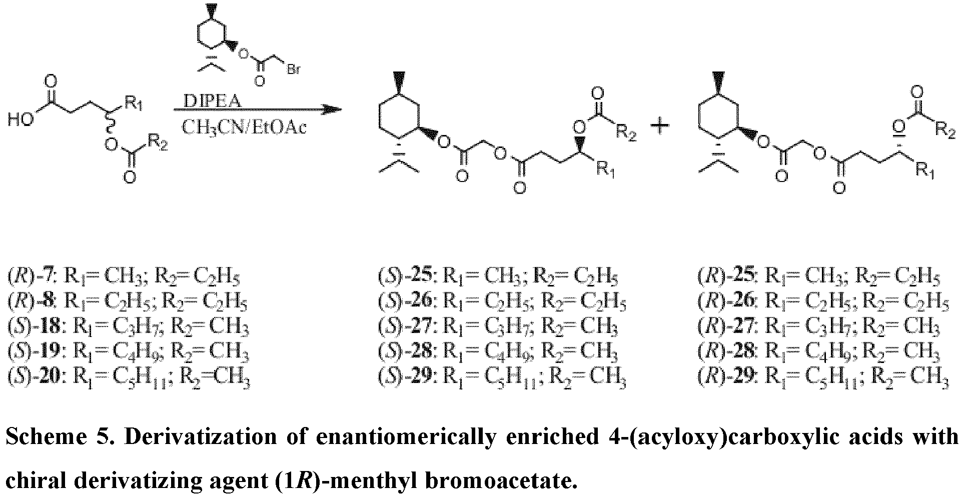

Characterization of the ester conjugates (*R*)-**25**/(*S*)-**25**: TLC: R_{f} = 0.35 (eluent: PE/EtOAc 10/1); ¹³C NMR (201 MHz, CDCl₃) δ 174.04 (Pr C-1), 172.39 (C-1), 167.41 (Oxo C-2), 75.62 (M C-1), 69.62 (C-4), 60.80 (Oxo C-1), 46.86 (M C-2), 40.63 (M C-6), 34.04 (M C-4), 31.33 (M C-5), 30.76 (C-3), 29.89 (C-2), 27.77 (Pr C-2), 26.14 (M C-7), 23.30 (M C-3), 21.93 (M C-10), 20.68 (M C-9), 19.88 (C-5), 16.21 (M C-8), 9.13 (Pr C-3). ¹H NMR (800 MHz, CDCl₃) δ 4.95 (h, *J* = 5x6.3 Hz, 1H, H-4), 4.76 (td, *J* = 2x 11.0, 4.5 Hz, 1H, M H-1), 4.60 and 4.58 (d, *J* = 15.8 Hz, 2H, (Oxo H-1), 2.49 and 2.45 (m, 2H, H-2), 2.30 (qm, *J* = 3x7.6 Hz, 2H, Pr H-2), 2.00 (m, 1H, M H-6), 1.94 and 1.93 (m, 2H, H-3), 1.84 (qqd, *J* = 3x7.0, 3x6.6, 2.6 Hz, 1H, M H-7), 1.68 (m, 2H, M H-3 and M H-4), 1.48 (m, 1H, M H-5), 1.38 (m, 1H, M H-2), 1.24 (d, *J* = 6.3 Hz, 3H, H-5), 1.13 (t, *J* = 7.6 Hz, 3H, Pr H-3), 1.06 (m, 1H, M H-3), 1.00 (td, *J* = 2×12.1, 11.1 Hz, 1H, M H-6), 0.91 (d, *J* = 6.6 Hz, 3H, M H-10), 0.89 (d, *J* = 7.0 Hz, 3H, M H-9), 0.86 (m, 1H, M H-4), 0.76 (d, *J* = 7.0 Hz, 3H, M H-8).

### Example 8. The synthesis of 2-(4-bromophenyl)-2-oxoethyl 4-(propionyloxy)pentanoate for HPLC stereochemical analysis of the target compound (R)-4-(propionyloxy)pentanoic acid.

2,4'-Dibromoacetophenone (55 mg; 0.2 mmol), 4-propanoyloxy valeric acid (52 mg; 0.3 mmol) and N,N-diisopropylethylamine (65 mg, 87 µL) in the mixture of EtOAc (4 mL) and CH₃CN (4 mL) were stirred for 3 hours at RT, until the conversion was complete by TLC. The mixture was evaporated and taken up in EtOAc (50 mL). After washing with saturated NaHCO₃ solution (2×10 mL) and brine (2×10 mL), drying over anhydrous Na₂SO₄, filtering, evaporation and flash chromatography (eluent: PE/EtOAc 10/1) the targetp-bromophenacyl ester **31** was gained in 94.2% yield (70 mg).

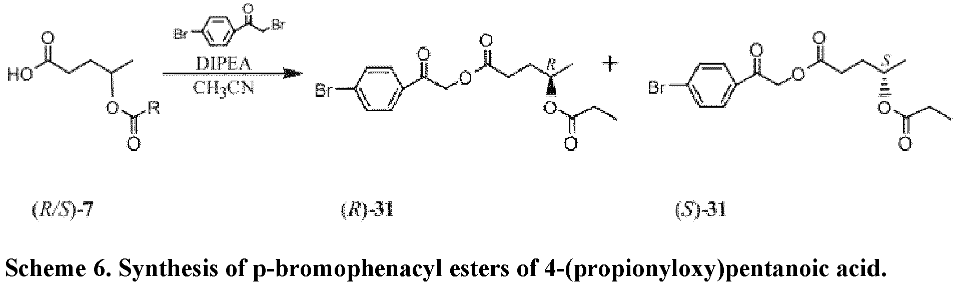

Characterization of the ester **31**: TLC: R_{f} = 0.43 (PE/EtOAc 10/3). ¹H NMR (800 MHz, CDCl₃) δ 7.78 (dm, *J* = 8.7 Hz, 2H, Ph 3,5), 7.65 (dm, *J* = 8.7 Hz, 2H, Ph 2,6), 5.33 and 5.29 (d, *J* = 16.3 Hz, 2H, Oxo-1), 4.99 (dqd, *J* = 7.6 3x6.3, 5.0 Hz, 1H, H-4), 2.57 and 2.54 (m, 2H, H-2), 2.32 (m, 2H, Pr-2), 1.97 (m, 2H, H-3), 1.26 (d, *J* = 6.3 Hz, 3H, H-5), 1.15 (tm, *J* = 2x7.6 Hz, 3H, Pr-3). ¹³C NMR (201 MHz, CDCl₃) δ 191.2 (Oxo-2), 174.1 (Pr-1), 172.5 (C-1), 132.8 (Ph-1), 132.2 (Ph-3,5), 129.2 (Ph-2,6), 129.1 (Ph-4), 69.6 (C-4), 65.8 (Oxo-1), 30.8 (C-3), 29.9 (C-2), 27.8 (Pr-2), 19.9 (C-5), 9.1 (Pr-3).

HPLC determination of *e.r.* - Column: CHIRALPAK^{®}IC (Daicel Chem. Ind-s Ltd.). Eluent: n-hexane/iPrOH 20%. Flow speed: 1 mL/min. Detector: UV, 254 nm. Retention times: (*R*)-**31**, 11.73 min; (*S*)-**31**, 9.60 min.

### References

1. D. M. Alonso, S. G. Wettstein and J. A. Dumesic, Green Chem., 2013, 15, 584-595. "Gamma-valerolactone, a sustainable platform molecule derived from lignocellulosic biomass".
2. I. T. Horváth, H. Mehdi, V. Fabos, L. Boda and L. T. Mika, Green Chem., 2008, 10, 238-242. "γ-Valerolactone - a sustainable liquid for energy and carbon-based chemicals."
3. J. Bozell, L. Moens, D. C. Elliott, Y. Wang, G. G. Neuenscwander, S. W. Fitzpatrick, R. J. Bilski and J. L. Jarnefeld, Resources Conservation and Recycling, 2000, 28(3-4): 227-239. "Production of levulinic acid and use as a platform chemical for derived products."
4. Z. Yu, X. Lu, J. Xiong, X. Li, H. Bai and N. Ji, ChemSusChem, 2020, 10.1002/cssc.202000175; "Heterogeneous Catalytic Hydrogenation of Levulinic Acid to γ-Valerolactone with Formic Acid as Internal Hydrogen Source: Key Issues and Their Effects"
5. S. Li, Y. Wang, Y. Yang, B. Chen, J. Tai, H. Liu and B. Han, Green Chem., 2019, 21, 770-774. "Conversion of levulinic acid to γ-valerolactone over ultra-thin TiO2 nanosheets decorated with ultrasmall Ru nanoparticle catalysts under mild conditions."
6. Z. Meng, Y. Liu, G. Yang, Y. Cao, H. Wang, F. Peng, P. Liu and H. Yu, ACS Sustainable Chem. Eng., 2019, 7, 16501-16510. "Electron-Rich Ruthenium on Nitrogen-Doped Carbons Promoting Levulinic Acid Hydrogenation to γ-Valerolactone: Effect of Metal-Support Interaction."
7. J. M. Tukacs, B. Fridrich, G. Dibó, E. Szekely and L. Mika, Green Chem., 2015, 17, 5189-5195. "Direct asymmetric reduction of levulinic acid to gamma-valerolactone: synthesis of a chiral platform molecule."
8. V. S. Shende, A. B. Raut, P. Raghav, A. A. Kelkar and B. M. Bhanage, ACS Omega, 2019, 4, 19491-19498. "Room-Temperature Asymmetric Transfer Hydrogenation of Biomass-Derived Levulinic Acid to Optically Pure γ-Valerolactone Using a Ruthenium Catalyst."
9. T. Osawa and Y. Tanabe, Catalysis Letters, 2018, 148(3), 824-830. "Facile Synthesis of Optically-Active γ-Valerolactone from Levulinic Acid and Its Esters Using a Heterogeneous Enantio-Selective Catalyst."
10. M. J. Rodriguez-Álvarez, N. Ríos-Lombardía, S. Schumacher, D. Pérez-Iglesias, F. Moris, V. Cadierno, J. Garcia-Alvarez and J. González-Sabín, ACS Catal. 2017, 7, 7753-7759. "Combination of Metal-Catalyzed Cycloisomerizations and Biocatalysis in Aqueous Media: Asymmetric Construction of Chiral Alcohols, Lactones, and γ-Hydroxy-Carbonyl Compounds."
11. K. Götz, A. Liese, M. Ansorge-Schumacher and L. Hilterhaus, Appl. Microbiol. Biotechnol., 2013, 97, 3865-3873. "A chemo-enzymatic route to synthesize (S)-γ-valerolactone from levulinic acid."
12. H. Jacobs, K. Berryman, J. Jones and A. Gopalan, Synth. Commun. 1990, 20, 999-1010. "Bakers' Yeast Reductions of Alkyl Levulinates: Synthesis of (R)-(+) and (S)-(-)4-methylbutyrolactones. "
13. E. V. Starodubtseva, O. V. Turova, M. G. Vinogradov, L. S. Gorshkova and V. A. Ferapontov, Russ. Chem. Bull., Intern. Ed., 2005, 54, 2374-2378. "Enantioselective hydrogenation of levulinic acid esters in the presence of the RuII-BINAP-HCl catalytic system."
14. T. Osawa and Y. Tanabe, Catal. Lett. 2018, 148, 824-830. "Facile Synthesis of Optically-Active γ-Valerolactone from Levulinic Acid and Its Esters Using a Heterogeneous Enantio-Selective Catalyst."
15. Y. Shimotori and T. Miyakoshi, J. Oleo Sci., 2006, 55, 629-635. "Synthesis of Optically Active γ-Lactones with Lipase Catalyst."
16. A.-B. L. Runmo, O. Pàmies, K. Faber and J.-E. Bäckvall, Tetrahedron Lett., 2002, 43, 2983-2986. "Dynamic kinetic resolution of γ-hydroxy acid derivatives."
17. P. Ferraboschi, P. Grisenti, A. Manzocchi and E. Santaniello, J. Chem. Soc., Perkin Trans. 1: Org. Bio-Org. Chem. (1972-1999), 1990, 9, 2469-2474. "Baker's yeast-mediated preparation of optically active aryl alcohols and diols for synthesis of chiral hydroxy acids."
18. M. T. Nuñez, V. S. Martin, Journal of Organic Chemistry, 1990, 55(6), 1928-32. "Efficient oxidation of phenyl groups to carboxylic acids with ruthenium tetraoxide. A simple synthesis of (R)-γ-caprolactone, the pheromone of Trogoderma granarium."
19. R. Datrika, R. S. Kallam, S. R. Khobare, V. S. Gajare, M. Kommi, M. H. Rama, V. Siddaiah and T. V. Pratap, Tetrahedron: Asymmetry, 2016, 27(14-15), 603-607. "Stereoselective synthesis of (R)-(-) and (S)-(+)-phoracantholide I from (R)-(+)-γ-valerolactone."
20. L. Orha, J. M. Tukacs, B. Gyarmati, A. Szilagyi, L. Kollar and L. T. Mika, ACS Sus. Chem. Eng., 2018, 6(4), 5097-5104. "Modular Synthesis of γ-Valerolactone-Based Ionic Liquids and Their Application as Alternative Media for Copper-Catalyzed Ullmann-type Coupling Reactions."
21. M. M. Enzelberger, U.T. Bornscheuer, I. Gatfield and R. D. Schmid, J. Biotechnol., 1997, 56, 129-133. "Lipase-catalysed resolution of gamma- and delta-lactones."

## Claims

1. An enzyme-catalysed method of stereoselectivity preparing gamma(acyloxy)carboxylic acids from a gamma-lactone wherein the process involves:
a) hydrolysis of the gamma-lactone to provide a gamma-hydroxycarboxylic acid salt;
b) acylation of the salt of step a) by incubation of the salt, an acyl donor and a lipase-catalyst in a solvent;
c) filtering, concentration, acidifying, extraction and acid-catalysed relactonization of the incubation mixture, to obtain a crude mixture of an enantiomerically enriched gamma(acyloxy)carboxylic acid and enantiomerically enriched gamma-lactone; and
d) purification.

2. The method according to claim 1, wherein the gamma-lactone is racemic gamma-lactone.

3. The method according to claim 1, wherein the gamma-lactone is enantiomerically enriched gamma-lactone.

4. The method according to claim 1, 2 or 3 wherein in step a) for hydrolysis of gamma-lactone is used alkali metal hydroxide.

5. The method according to claim 1, 2 or 3, wherein in step a) the gamma-hydroxy pentanoic acid salt is obtained by hydrolysing gamma-valerolactone.

6. The method according to claim 5, wherein the gamma-valerolactone is hydrolysed in an other solvent than ethanol.

7. The method according to claims 1-6, wherein in the incubation of the reaction mixture, with the aim of acylation of the salt an alternative temperature between cut-off values -84 °C and +70 °C is used.

8. The method according to claims 1-7, wherein in the acylation mixture an organic solvent, other than acetonitrile is used.

9. The method according to claims 1-8, wherein acyl donor used in the synthesis is an activated ester, such as vinyl ester with different acyl moieties.

10. The method according to claims 1-9, wherein Candida antarctica lipase B immobilized via interfacial activation on a resin which is macroporous support formed by poly(methyl methacrylate) crosslinked with divinylbenzene is used for the catalysis of the acyl transfer in the reaction.

11. The method according to claims 1-10, wherein sodium hydrogen sulphate water solution is used for the acidification of the reaction mixtures at the end of the incubation and thereafter, depending on the structure of the gamma-hydroxy carboxylic acid a catalytic amount of *para*-toluenesulfonic acid in toluene is used for relactonisation of the crude product.

12. The method according to claims 1-11, wherein at the end of the incubation the reaction mixture is filtered and evaporated to dryness and after that it is acidified with NaHSO₄ and extracted with ethyl acetate.

13. The method according to claims 1-12, wherein the product extracted from the reaction mixture after acidification with ethyl acetate is relactonised, if necessary, in toluol during 12 hours by stirring with catalytic amount of *para*-toluenesulfonic acid.

14. The method according to claims 1-13, wherein for separation of the target enantiomerically enriched gamma-(acyloxy)carboxylic acid and relactonized and also enantiomerically enriched gamma-lactone is used either column chromatography, extraction with NaHCO₃ water solution followed by acidification or distillation or any combination of two methods out of three listed above in this claim.
